# FASCICULE DE BREVET EUROPEEN

(11) **EP 0 692 247 B1**
(45) Date de publication et mention de la délivrance du brevet: **08.01.1997**
(21) Numéro de dépôt: 95401204.3
(22) Date de dépôt: 23.05.1995
(51) Int. Cl.: A61K 7/42, A61K 7/48, A61K 7/06

(54) **Composition à usage topique contenant un antioxydant et un extrait bactérien**
Topisches Mittel enthaltend einen Antioxidans und einem bakteriellen Extrakt
Topical composition containing an anti-oxidant and a bacterial extract

(30) Priorité: 16.06.1994 FR 9407397
(43) Date de publication de la demande: 17.01.1996
(73) Titulaire: L'OREAL, 75008 Paris (FR)
(72) Inventeur: De Rigal, Jean, F-77410 Claye Souilly (FR); Leveque, Jean-Luc, F-93340 Drancy (FR); Contamin, Jean-Claude, F-06340 Cantaron (FR); Aubert, Lucien, Les Rocailles, F-06320 Cap d'Ail (FR)
(74) Mandataire: Tezier Herman, Béatrice

(56) Documents cités:
- FR-A- 2 283 223
- FR-A- 2 693 654
- GB-A- 2 034 687

## Description

La présente invention a trait à une composition, cosmétique ou pharmaceutique, à appliquer sur la peau et/ou les cheveux, avant, pendant et/ou après exposition à un rayonnement UV, ladite composition étant susceptible de prévenir et/ou d'atténuer les dommages causés par ledit rayonnement.

Il est connu que les rayons lumineux de longueurs d'onde comprises entre 320 nm et 400 nm (UV-A) permettent le brunissement de l'épiderme humain; ils sont toutefois susceptibles d'induire une altération de celui-ci, notamment dans le cas d'une peau sensible ou d'une peau continuellement exposée. Les rayons UV-A provoquent en particulier une perte d'élasticité de la peau et l'apparition de rides conduisant à un vieillissement prématuré. Il est également connu que les rayons lumineux de longueurs d'onde comprises entre 280 et 320 nm (UV-B), provoquent des érythèmes et des brûlures cutanées qui peuvent nuire au développement du bronzage naturel.
Il est donc nécessaire, afin de conserver une qualité de la peau correcte après une exposition à un rayonnement UV, de la préparer avant l'exposition, de la protéger pendant l'exposition et d'éventuellement atténuer les dommages causés par l'exposition.

La présente invention a pour but de proposer une composition à appliquer sur la peau et/ou les cheveux, avant, pendant et/ou après exposition au rayonnement UV et susceptible de prévenir et/ou d'atténuer les dommages causés par ledit rayonnement.

Un objet de la présente invention est donc une composition à usage topique, en particulier pour la photoprotection de la peau et/ou des cheveux, comprenant, dans un support cosmétiquement acceptable, une substance active constituée de l'association d'au moins un agent antioxydant et d'au moins un extrait de bactéries filamenteuses non photosynthétiques et non fructifiantes.

Dans la suite de la présente description, les pourcentages sont donnés en poids sauf indication contraire.

La caractéristique des compositions selon l'invention est qu'elles comprennent, en tant que substance active, une association constituée d'au moins un agent antioxydant et d'au moins un extrait de bactéries filamenteuses non photosynthétiques et non fructifiantes.
On a en effet constaté que l'association de ces deux constituants bien particuliers permet l'obtention d'un effet amélioré dans le cadre de la photoprotection de la peau et/ou du cheveu.

Par extrait de bactéries filamenteuses non photosynthétiques et non fructifiantes, on entend aussi bien les extraits proprement dits que la biomasse obtenue après cultures desdites bactéries, ladite biomasse pouvant être partiellement déshydratée et/ou broyée.
Les extraits de bactéries selon l'invention sont préparés à partir de bactéries filamenteuses non photosynthétiques et non fructifiantes telles que définies selon la classification du Bergey's Manual of Systematic Bacteriology (vol. 3, section 23, 9°édition, 1989), parmi lesquelles on peut citer les bactéries appartenant à l'ordre des Beggiatoales, et plus particulièrement les bactéries appartenant aux genres Beggiatoa, Vitreoscilla, Flexithrix ou Leucothrix. Parmi les bactéries appartenant au genre Beggiatoa, on peut employer de préférence, les bactéries des souches de Beggiatoa alba (ATCC 33555).
Pour préparer l'extrait selon l'invention, on peut cultiver lesdites bactéries selon les méthodes connues de l'homme du métier, puis les séparer de la biomasse obtenue, par exemple par filtration, centrifugation et/ou lyophilisation.
On peut utiliser l'extrait sous la forme de dérivés, par exemple de dérivés au moins partiellement acylés. L'acylation peut alors être effectuée à l'aide d'anhydride d'acide organique carboxylique, tel que l'anhydride acétique, ou à l'aide du chlorure d'acide correspondant tel que le chlorure d'acétyle. L'acylation est effectuée de manière qu'au moins une partie des groupements amine primaire et secondaire présents dans la biomasse bactérienne soient sous forme acylée.
Dans les compositions selon l'invention, on peut utiliser 0,01 à 2% de matière sèche d'extrait de bactéries par rapport au poids total de la composition.
L'extrait de bactéries peut être incorporé sous forme de dispersion dans un véhicule approprié tel que l'eau, les solvants organiques, les corps gras y compris les huiles, seul ou en mélange.

La composition cosmétique selon l'invention comprend également un agent antioxydant. On entend par agent antioxydant tout composé susceptible d'avoir une action anti-radicaux libres oxygénés et/ou inactivatrice des peroxydes.
Ledit agent antioxydant est présent dans la composition à raison de 0,001 à 30% en poids, ladite quantité dépendant de la nature dudit antioxydant.

Ainsi, on peut par exemple utiliser un agent antioxydant choisi parmi
. le tocophérol et ses dérivés, entre autre acétate, linoléate ou nicotinate, de préférence à des concentrations de l'ordre de 0,1 à 5%,
. le γ-orizanol (0,1 à 5%),
. les pidolates de lysine ou d'arginine (0,5 à 10%),
. les extraits végétaux tels que l'extrait de mélisse (0,01 à 2%), l'extrait de silimarine (0,01 à 2%), l'extrait de gingko biloba (0,05 à 2%), l'extrait de sauge (0,05 à 2%), l'extrait de noix de cola (0,05 à 2%), l'extrait de rutine (0,1 à 2%) ou l'extrait de thym (0,1 à 2%), les % étant donnés en matière sèche,
. les oligomères proanthocyanolidiques de pin, d'aubépine ou de raisin (0,1 à 2%),
. le di-tertiobutyl-hydroxybenzylidène camphre (0,1 à 2%),
. le thé vert (0,1 à 2%),
. la caféine (0,1 à 5%),
. le glycérol (2 à 30%),
. le mannitol (2 à 30%),
. la carnosine (0,1 à 2%),
. la superoxyde dismutase (100 à 10 000 Ul/100 g),
. la guanosine (0,01 à 1%),
. les microalgues contenant de l'ethoxyquine telles que l'Hématococcus (0,005 à 1%),
. le pentasodium aminotriméthylène phosphonate (0,001 à 0,5%),
. la lactoperoxydase (0,01 à 0,1%) et
. la lactoferrine (0,01 à 0,1%).
On peut également utiliser un mélange de plusieurs antioxydants.

Les compositions selon l'invention peuvent se présenter sous toutes les formes envisageables dans le domaine cosmétique ou pharmaceutique, en particulier sous forme d'un produit de soin, d'un produit antisolaire, d'un produit après-soleil, d'un produit capillaire, ou d'un produit de maquillage.

Les compositions selon l'invention peuvent donc comprendre en outre au moins un système photoprotecteur classique capable de filtrer les rayons UV (UV-A et/ou UV-B) et qui peut donc être constitué par un ou plusieurs filtres organiques solaires (absorbeurs d'UV) et/ou par un ou plusieurs (nano)pigments minéraux à base d'oxydes métalliques, et en particulier d'oxyde de titane, agissant par blocage physique du rayonnement (réflecteurs et/ou diffuseurs d'UV),.

Les filtres solaires organiques classiques peuvent être choisis, seuls ou en mélanges, parmi l'acide 2-phényl benzimidazole 5-sulfonique et ses sels, les dérivés cinnamiques tels que par exemple le p-méthoxycinnamate de 2-éthylhexyle, les dérivés salicyliques comme par exemple le salicylate de 2-éthylhexyle et le salicylate d'homomenthyle, les dérivés du camphre comme par exemple le 3(4-méthylbenzylidène)camphre ou l'acide camphosulfonique (1,4 divinylbenzène) ainsi que ses dérivés (FR 2528420), les dérivés de triazine tels que la 2,4,6-tris [p-(2'-éthylhexyl-1'-oxycarbonyl)anilino] 1,3,5-triazine, les dérivés de la benzophénone tels que la 2-hydroxy 4-méthoxybenzophénone, les dérivés du dibenzoylméthane tels que le 4-tert-butyl 4'-méthoxydibenzoylméthane, les dérivés de β,β-diphénylacrylate tels que le α-cyano-β,β-diphénylacrylate de 2-éthylhexyle, les dérivés de l'acide p-aminobenzoïque comme par exemple le paradiméthylaminobenzoate d'octyle, l'anthranilate de menthyle, les polymères filtres et silicones filtres décrits dans la demande WO-93-04665. D'autres exemples de filtres organiques sont donnés dans la demande de brevet EP-A 0 487 404.

Le ou les filtres organiques sont généralement présents dans les compositions selon l'invention à une concentration comprise entre 0,1 et 30 % en poids, et de préférence entre 0,5 et 25 % en poids, par rapport au poids total de la composition.

Les oxydes métalliques constitutifs des pigments ou des nanopigments utilisables dans le cadre des compositions conformes à la présente invention peuvent être tous ceux déja connus en soi pour leur activité photoprotectrice. Ainsi, ils peuvent être notamment choisis, seuls ou en mélanges, parmi les oxydes de titane, de zinc, de fer, de zirconium, de cérium ou leurs mélanges.

De préférence, on met en oeuvre des nanopigments d'oxydes métalliques.

De tels nanopigments d'oxydes métalliques, enrobés ou non enrobés, sont des produits connus de l'homme de l'art et sont en particulier décrits dans la demande de brevet EP-A-0 518 773, dont l'enseignement est, à cet égard, inclus à titre de référence dans la présente description. A titre de nanopigments commerciaux complémentaires non cités dans la demande précitée mais également utilisables dans le cadre de la présente invention, on peut en outre mentionner les produits vendus sous les noms de marque UVT M 160, UVT M 212 et UVT M 262 par la Société KEMIRA, et MT 100 SA ou MT 100 SAS par la Société TAYCA.

La taille moyenne des particules primaires des nanopigments qui peuvent être présents dans les compositions selon l'invention est généralement comprise entre 5 nm et 100 nm, de préférence entre 10 et 50 nm.

Les nanopigments sont généralement présents dans les compositions selon l'invention à une concentration comprise entre 0,1 et 30 % en poids, et de préférence entre 1 et 20 % en poids, par rapport au poids total de la composition.

De préférence, la teneur global du système filtrant (filtre(s) organique(s) + (nano)pigment(s)) n'excède pas 40 % du poids total de la composition antisolaire finale.

Plus particulièrement, la composition selon l'invention peut comprendre en outre un filtre solaire comme par exemple ceux décrits dans le brevet FR 2528420 et préférentiellement le sel de triéthanolamine de l'acide terephtalylidène dicamphosulfonique ou MEXORYL SX ®.

Les compositions selon l'invention peuvent se présenter sous forme d'une émulsion, simple ou complexe (H/E, E/H, H/E/H ou E/H/E) telle qu'une crème, un lait, un gel, une pommade ou un gel crème, sous forme de poudre, de bâtonnet solide, ou encore sous forme de liquide, éventuellement conditionné en aérosol et se présentant sous forme de mousse ou de spray. Elles peuvent également se présenter sous forme de suspension ou de dispersion dans des solvants ou des corps gras, sous forme de dispersion vésiculaire non ionique, sous forme de shampooing, de lotion, de laque, ou encore sous forme solide ou pâteuse, anhydre ou aqueuse.
Les compositions de l'invention peuvent être préparées selon les techniques bien connues de l'homme du métier.

L'invention est illustrée plus en détail dans les exemples suivants.

### Exemple 1

On prépare une composition antisolaire ayant la composition suivante:

| | |
|---|---|
| . acide stéarique | 3,0% |
| . alcool cétylique | 2,0% |
| . monostéarate de glycérol autoémulsionnable | 2,5% |
| . vaseline | 2,0% |
| . huile de tournesol | 6,0% |
| . perhydrosqualène | 3,0% |
| . octylméthoxycinnamate | 4,0% |
| . sel de triéthanolamine de l'acide terephtalylidène dicamphosulfonique (Mexoryl SX) | 2,6% |
| . glycérol | 3,0% |
| . conservateurs | 0,3% |
| . superoxyde dismutase | 1200 Ul/100g |
| . pentasodium aminotriméthylène phosphonate | 0,1% |
| . tocophérol | 2,0% |
| . extrait de bactéries Beggiatoa alba | 0,05% |
| . eau | qsp 100% |

On obtient ainsi une composition permettant une bonne protection de la peau au rayonnement UV.

### Exemple 2

On prépare une composition après-soleil ayant la composition suivante:

| | |
|---|---|
| . alcool cétostearylique | 1,0% |
| . alcool cétylique | 2,0% |
| . monostéarate de glycérol autoémulsionnable | 3,0% |
| . beurre de karité | 3,0% |
| . huile de son de riz | 1,0% |
| . huile d'avocat | 2,0% |
| . di-octyl palmitate | 5,0% |
| . conservateurs | 0,3% |
| . cyclométhicone | 1,0% |
| . Carbomer 940 | 0,3% |
| . triéthanolamine | 0,3% |
| . acétate de tocophérol | 3,0% |
| . extrait de bactéries Beggiatoa alba | 0,1% |
| . eau | qsp 100% |

On obtient ainsi une composition après-soleil susceptible d'atténuer les dommages causés par le rayonnement UV.

### Exemple 3

On prépare une crème de soin ayant la composition suivante:

| | |
|---|---|
| . acide stéarique | 3,0% |
| . alcool cétylique | 2,0% |
| . monostéarate de glycérol autoémulsionnable | 3,0% |
| . octylméthoxycinnamate | 2,0% |
| . glycérol | 5,0% |
| . conservateurs | 0,3% |
| . superoxyde dismutase | 1000 Ul/100g |
| . tocophérol | 2,0% |
| . extrait de bactéries Beggiatoa alba | 0,05% |
| . Carbomer 934 | 0,2% |
| . triéthanolamine | 0,2% |
| . eau | qsp 100% |

On obtient ainsi une composition permettant une bonne protection de la peau.

## Revendications

1. Composition à usage topique, en particulier pour la photoprotection de la peau et/ou des cheveux, comprenant, dans un support cosmétiquement acceptable, une substance active constituée de l'association d'au moins un agent antioxydant et d'au moins un extrait de bactéries filamenteuses non photosynthétiques et non fructifiantes.

2. Composition selon la revendication 1, dans laquelle lesdites bactéries appartiennent à l'ordre des Beggiatoales.

3. Composition selon la revendication 2, dans laquelle lesdites bactéries sont choisies parmi les bactéries appartenant aux genres Beggiatoa, Vitreoscilla, Flexithrix ou Leucothrix.

4. Composition selon la revendication 3, dans laquelle les bactéries sont choisies parmi des souches de Beggiatoa alba.

5. Composition selon l'une quelconque des revendications précédentes, dans laquelle l'agent antioxydant est choisi, seul ou en mélange, dans le groupe constitué par le tocophérol et ses dérivés; le γ-orizanol; les pidolates de lysine ou d'arginine; les extraits de mélisse, de silimarine, de ginkgo biloba, de sauge, de noix de cola, de rutine ou de thym; les oligomères proanthocyanolidiques de pin, d'aubépine ou de raisin; le di-tertiobutylhydroxybenzylidène camphre; le thé vert; la caféine; le glycérol; le mannitol; la carnosine; la superoxyde dismutase; la guanosine; les microalgues contenant de l'ethoxyquine; le pentasodium-aminotriméthylène-phosphonate; la lactoperoxydase et la lactoferrine.

6. Composition selon l'une quelconque des revendications précédentes, dans laquelle l'agent antioxydant est présent à raison de 0,001 à 30 % en poids par rapport au poids de la composition.

7. Composition selon l'une quelconque des revendications précédentes, dans laquelle l'extrait de bactéries est présent à raison de 0,01 à 2 % en poids par rapport au poids de la composition.

8. Composition selon l'une quelconque des revendications précédentes, à appliquer sur la peau et/ou les cheveux avant, pendant et/ou après une exposition à un rayonnement UV, en particulier solaire.

9. Composition selon l'une quelconque des revendications précédentes, contenant au moins un système photoprotecteur classique capable de filtrer les rayons UV (UV-A et/ou UV-B).

10. Composition selon la revendication 9, contenant un sel de triéthanolamine de l'acide terephtalylidène dicamphosulfonique.

11. Utilisation d'une association d'un agent antioxydant et d'au moins un extrait de bactéries filamenteuses non photosynthétiques et non fructifiantes comme substance active dans une composition cosmétique destinée à la photoprotection de la peau et/ou des cheveux.

12. Utilisation d'une association d'un agent antioxydant et d'au moins un extrait de bactéries filamenteuses non photosynthétiques et non fructifiantes comme substance active pour la préparation d'une composition pharmaceutique destinée à la photoprotection de la peau et/ou des cheveux.

## Claims

1. Composition for topical use, in particular for the photoprotection of the skin and/or the hair, comprising, in a cosmetically acceptable support, an active substance consisting of the combination of at least one antioxidant and at least one extract of non-photosynthetic and non-fruiting filamentous bacteria.

2. Composition according to Claim 1, in which the said bacteria belong to the order of Beggiatoales.

3. Composition according to Claim 2, in which the said bacteria are chosen from bacteria belonging to the genera Beggiatoa, Vitreoscilla, Flexithrix or Leucothrix.

4. Composition according to Claim 3, in which the bacteria are chosen from the strains of Beggiatoa alba.

5. Composition according to any one of the preceding claims, in which the antioxidant is chosen, alone or as a mixture, from the group consisting of tocopherol and derivatives thereof; γ-orizanol; lysine pidolate or arginine pidolate; extracts of balm, of silimarin, of ginkgo biloba, of sage, of cola nuts, of rutin or of thyme; proanthocyanolidine oligomers of pine, of hawthorn or of grape; di-tert-butylhydroxybenzylidene-camphor; green tea; caffeine; glycerol; mannitol; carnosine; superoxide dismutase; guanosine; microalgae containing ethoxyquine; pentasodium aminotrimethylenephosphonate; lactoperoxidase and lactoferrin.

6. Composition according to any one of the preceding claims, in which the antioxidant is present at an amount of from 0.001 to 30% by weight relative to the weight of the composition.

7. Composition according to any one of the preceding claims, in which the bacterial extract is present at an amount of from 0.01 to 2% by weight relative to the weight of the composition.

8. Composition according to any one of the preceding claims to be applied to the skin and/or the hair before, during and/or after exposure to UV radiation, in particular solar UV radiation.

9. Composition according to any one of the preceding claims, containing at least one conventional photoprotective system capable of screening UV (UV-A and/or UV-B) rays.

10. Composition according to Claim 9, containing a triethanolamine salt of terephthalylidenedicamphosulphonic acid.

11. Use of a combination of an antioxidant and at least one extract of non-photosynthetic and non-fruiting filamentous bacteria as active substance in a cosmetic composition intended for the photoprotection of the skin and/or the hair.

12. Use of a combination of an antioxidant and at least one extract of non-photosynthetic and non-fruiting filamentous bacteria as active substance for the preparation of a pharmaceutical composition intended for the photoprotection of the skin and/or the hair.

## Patentansprüche

1. Zusammensetzung zur topischen Anwendung, insbesondere zum Lichtschutz der Haut und/oder der Haare, die in einem kosmetisch akzeptablen Träger eine Wirksubstanz enthält, die aus einer Kombination mindestens eines Anitioxidationsmittels und mindestens eines Extrakts von Filamente bildenden nicht phototrophen und nicht sporenbildenden Bakterien besteht.

2. Zusammensetzung nach Anspruch 1, in der die Bakterien zur Ordnung Beggiatoales gehören.

3. Zusammensetzung nach Anspruch 2, in der die Bakterien ausgewählt sind unter Bakterien, die zu den Gattungen Beggiatoa, Vitreoscilla, Flexithrix oder Leucothrix gehören.

4. Zusammensetzung nach Anspruch 3, in der die Bakterien ausgewählt sind unter Stämmen von Beggiatoa alba.

5. Zusammensetzung nach einem der vorhergehenden Ansprüche, in der das Antioxidationsmittel alleine oder im Gemisch ausgewählt ist unter Tocopherol und seinen Derivaten, γ-Orizanol, Pidolaten von Lysin oder Arginin, Melissenextrakt, Extrakt von Silymarin, Extrakt von Ginkgo biloba, Salbeiextrakt, Kolanußextrakt, Extrakt von Rutin oder Thymian, Proanthocyanolid-Oligomeren von Kiefer, Weißdorn oder Trauben, Di-tert.-butylhydroxybenzylidencampher, grünem Tee, Coffein, Glycerin, Mannit, Carnosin, Superoxid-Dismutase, Guanosin, Mikroalgen, die Ethoxyquin enthalten, Pentanatriumaminotrimethylenphosphonat, Lactoperoxidase und Lactoferrin.

6. Zusammensetzung nach einem der vorhergehenden Ansprüche, in der das Antioxidationsmittel in Anteilen im Bereich von 0,001 bis 30 Gew.-%, bezogen auf das Gewicht der Zusammensetzung, vorliegt.

7. Zusammensetzung nach einem der vorhergehenden Ansprüche, in der der Bakterienextrakt in Anteilen von 0,01 bis 2 Gew.-%, bezogen auf das Gewicht der Zusammensetzung, vorliegt.

8. Zusammensetzung nach einem der vorhergehenden Ansprüche zur Anwendung auf die Haut und/oder das Haar vor, während und/oder nach einer Exposition gegenüber UV-Strahlung und insbesondere Sonnenstrahlung.

9. Zusammensetzung nach einem der vorhergehenden Ansprüche, die mindestens ein herkömmliches Lichtschutzsystem enthält, das UV (UV-A und/oder UV-B)-Strahlung filtern kann.

10. Zusammensetzung nach Anspruch 9, die ein Triethanolaminsalz von Terephthalylidendicamphosulfonsäure enthält.

11. Verwendung einer Kombination eines Antioxidationsmittels und mindestens eines Extrakts von Filamente bildenden nicht phototrophen und nicht sporenbildenden Bakterien als Wirksubstanz in einer kosmetischen Zusammensetzung, die zum Lichtschutz der Haut und/oder der Haare bestimmt ist.

12. Verwendung einer Kombination eines Antioxidationsmittels und mindestens eines Extrakts von Filamente bildenden nicht phototrophen und nicht sporenbildenden Bakterien als Wirksubstanz zur Herstellung einer pharmazeutischen Zusammensetzung, die zum Lichtschutz der Haut und/oder der Haare bestimmt ist.
